# EUROPEAN PATENT APPLICATION

(11) **EP 4 434 523 A1**
(43) Date of publication of application: **25.09.2024**
(21) Application number: 23163445.2
(22) Date of filing: 22.03.2023
(51) Int. Cl.: A61K 31/453, A61P 37/00

(54) **A CYCLIN-DEPENDENT KINASE 2/4/9 INHIBITOR FOR THE TREATMENT OF NEUTROPENIA**

(71) Applicant: Eberhard Karls Universität Tübingen, Medizinische Fakultät, 72074 Tübingen (DE)
(72) Inventor: Skokowa, Julia, 72127 Kusterdingen (DE); Nasri, Masoud, 72074 Tübingen (DE); Dannenmann, Benjamin, 75387 Neubulach (DE); Doll, Larissa, 72070 Tübingen (DE)
(74) Representative: Witte, Weller & Partner Patentanwälte mbB

(57) **Abstract**

The invention relates to a new agent for the prophylaxis and/or treatment of neutropenia, a pharmaceutical composition comprising said agent, and to a method for the prophylactic and/or therapeutic treatment of neutropenia.

## Description

### FIELD OF THE INVENTION

The invention relates to a new agent for the prophylaxis and/or treatment of neutropenia, a pharmaceutical composition comprising said agent, and to a method for the prophylactic and/or therapeutic treatment of neutropenia.

### BACKGROUND OF THE INVENTION

Neutropenia is a disorder characterized by an abnormally low concentration of neutrophils in the blood and bone marrow. Neutrophils make up the majority of circulating white blood cells and serve as the primary defense against infections by destroying bacteria, bacterial fragments and immunoglobulin-bound viruses in the blood. People with neutropenia are more susceptible to bacterial infections and, without prompt medical attention, the condition may become life-threatening (neutropenic sepsis). Neutropenia can be acute (temporary) or chronic (long lasting). The term is sometimes used interchangeably with "leukopenia" ("deficit in the number of white blood cells").

Neutropenia can be divided into acquired and congenital. The two main types of the congenital condition are severe congenital neutropenia (CN or SCN) and cyclic neutropenia (CyN).

Cyclic neutropenia is characterized by fluctuating neutrophil counts from normal levels to zero while severe congenital neutropenia is characterized by very low absolute neutrophil count (ANC) (500/ml) observed at birth, maturation arrest of the myelopoiesis in bone marrow at the promyelocyte/myelocyte stage, and early onset of bacterial infections.

Severe congenital neutropenia (CN) is associated with mutations in various genes such as *ELANE, HAX1, JAGN1, SBDS* and others. It may be diagnosed by measuring a very low ANC in the blood and examining bone marrow aspirate to identify myeloid maturation arrest.

CN is usually diagnosed shortly after birth, while diagnosis for CyN is generally raised at different ages, and the main clinical manifestation is recurrent acute stomatologic disorders. Bone marrow examination is often necessary to rule out malignant transformation of hematopoiesis, determine cellularity, assess myeloid maturation, and detect signs of a precise etiology, with cytogenetic bone marrow studies now crucial when CN/SCN is suspected. Antineutrophil antibody assay, immunoglobulin assay (Ig GAM), lymphocyte immunophenotyping, pancreatic markers (serum trypsinogen and fecal elastase) and liposoluble vitamin levels (vitamins A, E and D) are also of interest in assessing CN/SCN and CyN.

Daily subcutaneous administration of recombinant human granulocyte colony stimulating factor (rhG-CSF) is the treatment of choice and leads to a substantial increase in absolute cell counts, a reduction of infections and a drastic improvement of quality of life. Although the majority of patients respond to daily treatment with rhG-CSF, approximately 15 % of patients do not respond to this cytokine at doses up to 50 µg/kg/day, and approximately 20 % of patients develop myelodysplasia (MDS) or acute myeloid leukemia (AML). Bone marrow transplantation is the only curative therapy for CN and it carries risks of severe adverse events,such as graft-versus-host disease and graft failure, therefore there is an urgent need for alternative therapeutic modalities for CN patients.

Against this background, the object of the present invention is to provide a new therapeutic and drug intervention for the treatment and prophylaxis of neutropenia. In particular, an active agent or compound is to be made available which mitigates and preferably even avoids the problems of the current treatment options for neutropenia.

The present invention satisfies these and other needs.

### SUMMARY OF THE INVENTION

The present invention provides a cyclin-dependent kinase 2/4/9 (CDK2/4/9) inhibitor and salts thereof, solvates thereof and solvates of the salts thereof, for use in the prophylaxis and/or treatment of neutropenia.

Cyclin-dependent kinases (CDKs) are members of the CDK family. CDK family members are highly similar to the gene products of S. *cerevisiae* cdc28, and *S*. *pombe* cdc2, and known as important cell cycle regulators. CDKs were first discovered for their role in regulating the cell cycle. They are also involved in regulating transcription, mRNA processing, and the differentiation of nerve cells. They are present in all known eukaryotes, and their regulatory function in the cell cycle has been evolutionarily conserved.

Cyclin-dependent kinase 2 or CDK2 associates with and is regulated by the regulatory subunits of the complex including cyclin E or A. Cyclin E binds G1 phase Cdk2, which is required for the transition from G1 to S phase while binding with Cyclin A is required to progress through the S phase. Its activity is also regulated by phosphorylation. Multiple alternatively spliced variants and multiple transcription initiation sites of this gene have been reported.

Cyclin-dependent kinase 4 or CDK4 is controlled by the regulatory subunits D-type cyclins. This kinase was shown to be responsible for the phosphorylation of retinoblastoma gene product (Rb). Cyclin D-CDK4 complexes are major integrators of various mitogenic and antimitogenic signals.

Cyclin-dependent kinase 9 or CDK9 is a cyclin-dependent kinase associated with P-TEFb. This kinase was found to be a component of the multiprotein complex TAK/P-TEFb, which is an elongation factor for RNA polymerase II-directed transcription and functions by phosphorylating the C-terminal domain of the largest subunit of RNA polymerase II. This protein forms a complex with and is regulated by its regulatory subunit cyclin T or cyclin K.

CDK2/4/9 inhibitors are molecules which specifically inhibit the activity of CDK2/4/9, either, in an embodiment, non-complexed or, in another embodiment, in complex with their respective regulatory subunits. According to the invention, a CDK2/4/9 inhibitor is understood to be a compound that, in one embodiment, inhibits CDK2 or CDK4 or CDK9, i.e., referring to three different inhibitors, such as a CDK2 inhibitor, CDK4 inhibitor, and a CDK9 inhibitor. In contrast, in another embodiment, it is understood to mean such a compound that inhibits all of CDK 2 and CDK4 and CDK9, but not necessarily all three CDKs to the same extent, i.e., referring to one inhibitor. The CDK-specific inhibitory effects can, if necessary, also occur in a concentration-dependent manner. That is, in one embodiment, a compound can inhibit only one or two of CDK2, CDK4, and CDK9 at low levels, while, in another embodiment, it can inhibit all three CDKs at higher levels, if necessary.

CDK2/4/9 inhibitors are often, but not exclusively, small molecules. However, they also include inhibitor proteins named by the small letter "p" followed by their molecular weight in kilodaltons, such as p15, p16, p18, p19, p21, p27, and p57.

The CDK2/4/9 inhibitor may exist in stereoisomeric forms (enantiomers, diastereomers). The invention therefore also encompasses the enantiomers or diastereomers and respective mixtures thereof. The stereoisomerically uniform constituents can be isolated in a known manner from such mixtures of enantiomers and/or diastereomers. If the CDK2/4/9 inhibitor may occur in tautomeric forms, the present invention encompasses all tautomeric forms.

Salts preferred for the purposes of the present invention are physiologically or pharmaceutically acceptable salts of the CDK2/4/9 inhibitor. Also encompassed, however, are salts which are themselves not suitable for pharmaceutical applications but can be used for example for the isolation or purification of the CDK2/4/9 inhibitor.

Examples of pharmaceutically acceptable salts of the CDK2/4/9 inhibitor include salts of inorganic bases like ammonium salts, alkali metal salts, in particular sodium or potassium salts, alkaline earth metal salts, in particular magnesium or calcium salts; salts of organic bases, in particular salts derived from cyclohexylamine, benzylamine, octylamine, ethanolamine, diethanolamine, diethylamine, triethylamine, ethylenediamine, procaine, morpholine, pyrroline, piperidine, N-ethylpiperidine, N-methylmorpholine, piperazine as the organic base; or salts with basic amino acids, in particular lysine, arginine, ornithine and histidine.

Examples of pharmaceutically acceptable salts of the CDK2/4/9 inhibitor also include salts of inorganic acids like hydrochlorides, hydrobromides, sulfates, phosphates or phosphonates; salts of organic acids, in particular acetates, formates, propionates, lactates, citrates, fumarates, maleates, benzoates, tartrates, malates, methanesulfonates, ethanesulfonates, toluenesulfonates or benzenesulfonates; or salts with acidic amino acids, in particular aspartate or glutamate.

Examples of pharmaceutically acceptable salts of the CDK2/4/9 inhibitor also include salts of inorganic acids like hydrochlorides, hydrobromides, sulfates, phosphates or phosphonates; salts of organic acids, in particular acetates, formates, propionates, lactates, citrates, fumarates, maleates, benzoates, tartrates, malates, methanesulfonates, ethanesulfonates, toluenesulfonates or benzenesulfonates; or salts with acidic amino acids, in particular aspartate or glutamate.

Solvates for the purposes of the invention refer to those forms of the CDK2/4/9 inhibitor, which in the solid or liquid state form a complex by coordination with solvent molecules. Hydrates are a specific form of solvates in which the coordination takes place with water.

CDK9 and some of its inhibitors are described in the review article of Anshabo et al. (2021), CDK9: A Comprehensive Review of Its Biology, and Its Role as a Potential Target for Anti-Cancer Agents, Front. Oncol. 11:678559. doi: 10.3389/fonc.2021.678559, CDK9 inhibitors which are suitable for carrying out the present invention are especially described in Supplementary Table 1.

According to the invention, "prophylaxis" and "treatment" of neutropenia are understood to mean any measure which at least mitigates or possibly even prevents a living being from developing or suffering from neutropenia and/or its symptoms. In particular, this is understood to include the prevention or reversal of the reduction of neutrophil granulocytes or neutrophils in the blood of a living being.

As used herein, the terms "living being" and "subject" can be used interchangeably. They refer to any kind of living creature, including a vertebrate, a mammal such as mouse, rat, cow, pig, goat, chicken, dog, monkey, horse or human. Preferentially, the living being is a human. The subject may be a subject suffering from neutropenia (a patient), but the subject may be also a healthy subject.

The finding of the inventors that CDK2/4/9 inhibitors can be used for prophylaxis and treatment of neutropenia was surprising and not to be expected. So far, most of the CDK2/4/9 inhibitors are only described for their uses in the treatment of solid and hematological malignancies, especially CDK9 inhibitors; see Anshabo et al. (*op. cit.*).

Indeed, prior art describes that treatment of endometrial carcinoma with a compound named flavopiridol (alvocidib), so far referred to as a CDK9 inhibitor, could even cause neutropenia as a side effect; see Grendys et al. (2005), A phase II evaluation of flavopiridol as second-line chemotherapy of endometrial carcinoma: a Gynecologic Oncology Group study, Gynecol. Oncol. 98(2), pages 249-53. A skilled person would therefore not consider the use of a CDK2/4/9 inhibitor, much less a CDK9 inhibitor, for the prophylaxis and treatment of neutropenia, since he/she would fear precisely the initiation or exacerbation of such a clinical picture. The inventors, on the other hand, could not confirm the putative observations of Grendys et al. On the contrary.

The inventors have found, both in an established and well-recognized *in vitro* model and *in vivo* in animal models of neutropenia and also in hematopoietic stem and progenitor cells from patients with severe congenital neutropenia, that treatment or administration of a CDK2/4/9 inhibitor results in significant and impressive improvement in granulopoiesis and clinical picture in patients.

The starting point for the inventors was the finding that flavopiridol (also known as alvocidib) can inhibit not only CDK9, as previously assumed, but also CDK2 and CDK4. In particular, in an exemplary experimental setup the inventors were able to demonstrate that the administration of flavopiridol enabled neutrophilic maturation of primary hematopoietic stem and progenitor cells and induced pluripotent stem cells of severe congenital neutropenia patients. Flavopiridol also further increased neutrophilic maturation of primary hematopoietic stem and progenitor cells of healthy control.

Along with its therapeutic effect, particularly advantageous thereby is the prophylactic effect of the CDK2/4/9 inhibitor for the prevention of neutropenia established by the inventors. Thus, the inventors surprisingly found that administration of the CDK2/4/9 inhibitor even to healthy individuals promotes the maturation of neutrophils. The fact that granulocytic differentiation of healthy donors' hematopoietic stem cells towards neutrophils is also promoted further supports the advantageousness of the application of the CDK2/4/9 inhibitor for the treatment of acquired neutropenia.

Surprisingly, the beneficial effects of the CDK2/4/9 inhibitor observed by the inventors occur independently of the mutations causing neutropenia, e.g., in CN the mutated genes *ELANE, HAX1, JAGN1, SRP54* etc. The new agent is therefore characterized by a particularly broad spectrum of application and a particularly large target group to be addressed.

Incidentally, the effects of the CDK2/4/9 inhibitor observed according to the invention are already apparent at low concentrations, whereas in the prior art, CDK inhibitors are usually applied at very high concentrations for the known indications. Whereas in the prior art for the clinical trials there, steady-state blood plasma levels of about 350 nM flavopiridol are used to inhibit CDKs and induce apoptosis in leukemia cells (Gerson et al. (2018), Flavopiridol - Pharmacology and molecular mechanisms of antineoplastic agents for hematologic malignancies, in 'Hematology', 7th Edition), the inventors see the positive effects with respect to neutropenia already at a fraction of the blood plasma concentrations used there, for example at about 1/9 of the published dose. The low concentrations of the compound according to the invention create therapeutic safety by minimizing the risk of side effects.

These findings by the inventors are therefore particularly beneficial because they now allow, for the first time, the treatment of such neutropenia patients who would not respond to the administration of rhG-CSF or who would develop myelodysplasia (MDS) or acute myeloid leukemia (AML), without the need for bone marrow transplantation.

In an embodiment of the invention the CDK2/4/9 inhibitor is selected from the group consisting of: flavopiridol (alvocidib), rohitukine, TP-1287, seliciclib (roscovitine/CYC202), dinaciclib (SCH 727965), SNS 032, P276-00, AT7519, voruciclib, CDKI-73, TG02, BAY1 143572 (atuveciclib), BAY1251152, AZD4573, i-CDK9, and NVP-2.

This measure has the advantage that CDK9 inhibitors are largely employed that are already in clinical or at least preclinical testing and therefore many details are already known about the efficacy profile, pharmacodynamics and pharmacokinetics, and possibly also about the side effect profile. Conversion into an operational therapeutic can therefore take place rapidly.

For example, flavopiridol, also known as alvocidib, is a flavonoid alkaloid CDK9 kinase inhibitor under clinical development for the treatment of acute myeloid leukemia. It has been studied also for the treatment of arthritis [Sekine et al. (2008), Successful treatment of animal models of rheumatoid arthritis with small-molecule cyclin-dependent kinase inhibitors, J. Immunol. 180 (3), pp. 1954-61] and atherosclerotic plaque formation [Ruel et al. (1999), Flavopiridol inhibits smooth muscle cell proliferation in vitro and neointimal formation in vivo after carotid injury in the rat, Circulation. 100 (6), pp. 659-65]. The target of alvocidib is the positive transcription elongation factor P-TEFb. Treatment of cells with alvocidib has been described to lead to inhibition of P-TEFb and the loss of mRNA production. The FDA has granted orphan drug designation to alvocidib in 2014 for the treatment of patients with acute myeloid leukemia.

In another embodiment of the invention said neutropenia is any selected from the group consisting of: congenital and acquired neutropenia, including severe congenital neutropenia (CN/SCN), cyclic neutropenia (CyN), chronic neutropenia, including idiopathic neutropenia, drug-induced neutropenia, immune neutropenia, acute neutropenia caused by chemotherapy or irradiation.

This measure has the advantage of adapting the invention to the treatment of such neutropenias, which occur frequently and for which no satisfactory therapeutic options exist to date for a significant proportion of affected patients.

In a preferred embodiment of the invention the CDK2/4/9 inhibitor is flavopiridol (alvocidib) and the neutropenia is severe congenital neutropenia (CN/SCN) or cyclic neutropenia (CyN).

In this embodiment, the knowledge gained by the inventors in the exemplary experiments is put into practice in an advantageous manner. Thus, using flavopiridol as an example and established experimental models of CN/SCN, the inventors have found that neutropenia can be successfully and effectively treated or prevented by means of CDK2/4/9 inhibitors.

In still another embodiment of the invention the CDK2/4/9 inhibitor is used at a dosage which, after administration into a living being, results in blood plasma concentrations which are in the area of approx. 10 - approx. 300, preferably approx. 20 - approx. 200, further preferably approx. 30 - approx. 100, and most preferably approx. 40 nM.

This measure has the advantage that sufficiently high concentrations of the CDK2/4/9 inhibitor are provided to achieve the curative and prophylactic effects with respect to neutropenia according to the invention. On the other hand, however, such high concentrations are not achieved to induce side effects as described in the prior art. For example, significantly increased CDK inhibitor concentrations are used for anti-tumor treatment (cf. Gerson et al., op. cit.), which, however, can be accompanied by significant side effects; cf. Grendys et al., op. cit.

In another embodiment of the invention the CDK2/4/9 inhibitor is used is in the prophylaxis and/or treatment of neutropenia patients who do not respond or do not respond satisfactorily to treatment with granulocyte colony-stimulating factor (G-CSF), preferably recombinant human G-CSF (rhG-CSF).

The advantage of this measure is that it can help the not insignificant proportion of patients who do not respond or do not respond sufficiently to the current standard therapy and would otherwise have to undergo risky bone marrow therapy. This is where the invention provides an effective remedy.

Although the CDK2/4/9 inhibitor can be used as a stand-alone agent or the only active agent. Therefore, in yet another embodiment of the invention the CDK9 inhibitor is used in combination with G-CSF, preferably recombinant human G-CSF (rhG-CSF).

This embodiment of the invention has the advantage that the current standard therapy in such patients who are susceptible to G-CSF is significantly improved and, if necessary, the amounts of G-CSF can be reduced because of the effect enhancement or synergistic effect, respectively. This also significantly reduces the risk of occurrence of side effects, such as MDS or AML.

Another subject-matter of the invention is a pharmaceutical composition for use in the prophylaxis and/or treatment of neutropenia comprising the CDK2/4/9 inhibitor and the salts thereof, the solvates thereof and the solvates of the salts thereof according to the invention, and a pharmaceutically acceptable carrier.

The features, properties, advantages and embodiments disclosed above and below for the CDK2/4/9 inhibitor according to the invention apply equally and *mutatis mutandis* to the pharmaceutical composition.

"Pharmaceutically acceptably carriers" are well known to the skilled person. They allow a proper formulation of the CDK2/4/9 inhibitor according to the invention and, if applicable, an additional active agent, such as G-CSF, and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cottonseed oil, safflower oil, sesame oil, olive oil, corn oil and soybean oil; glycols such as propylene glycol; esters, such as ethyl oleate and ethyl laurate; agar; buffering agents, such as magnesium hydroxide and aluminum hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The pharmaceutical composition may comprise the CDK2/4/9 inhibitor and, if applicable, an additional active agent, such as G-CSF, in "effective amounts". An "effective amount" (or, "therapeutically effective amount") is an amount sufficient to effect a beneficial or desired clinical result upon treatment. An effective amount can be administered to a subject or living being in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically considered when determining an appropriate dosage to achieve an effective amount. These factors include age, sex and weight of the subject, the condition being treated, and the severity of the condition.

A further subject-matter of the present invention is a method for the prophylactic and/or therapeutic treatment of neutropenia in a living being, comprising the administration of an effective amount of the CDK2/4/9 inhibitor and the salts thereof, the solvates thereof and the solvates of the salts thereof according to the invention, or the pharmaceutical composition of the invention to said living being.

The features, properties, advantages and embodiments disclosed above and below for the CDK9 inhibitor and the pharmaceutical according to the invention apply equally and *mutatis mutandis* to the method according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

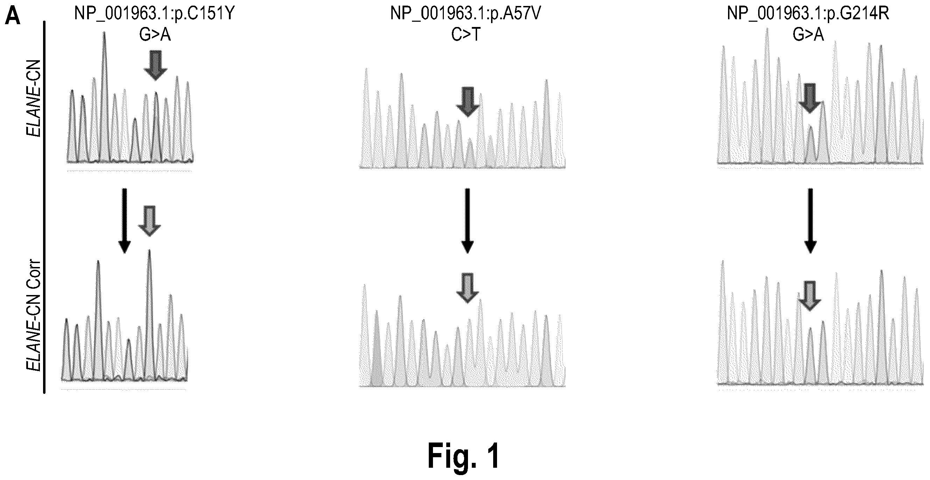

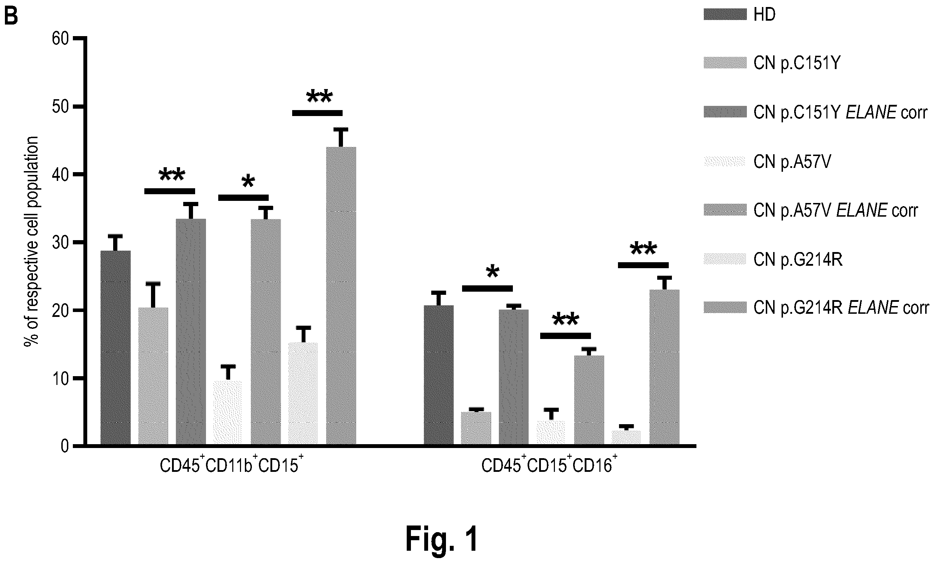

- Fig. 1:: Generation of isogenic iPSC lines using CRISPR/Cas9 gene editing. A. Sanger sequencing traces from ELANE-CN patient-derived iPSCs with p. C151Y, p. A57V, and p. G214R mutations, before and after CRISPR/Cas9 based mutation correction. B. Flow cytometry analysis of suspension cells harvested from EB culture on day 28 of differentiation of ELANE-CN iPSC and ELANE-CN corr lines. Data represent means ± SD from two independent experiments, each in duplicates, *P < 0.05, **P < 0.01.

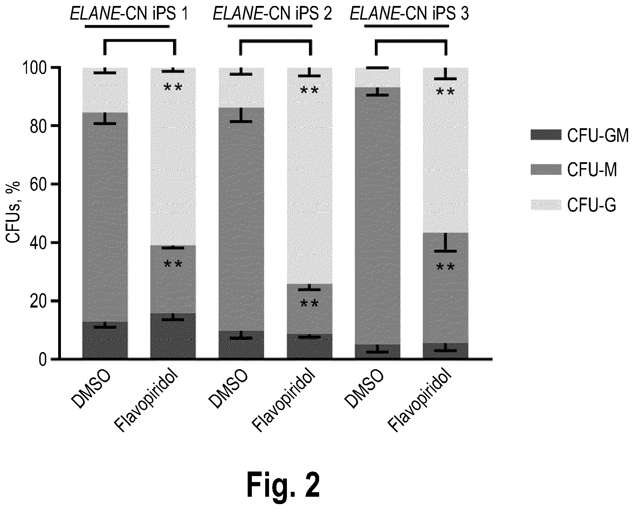

- Fig. 2:: Flavopiridol treatment restored granulocytic differentiation of ELANE-CN iPSCs. Colony-forming unit (CFU) assay of CD34⁺ cells harvested from EB-based CN-iPSC culture on day 14 of differentiation treated with eleven short listed small molecules. Data represent means ± SD from two independent experiments., **P < 0.01. CFU assay of CD34⁺ cells harvested from EB-based three CN-iPSCs culture on day 14 of differentiation treated with 40 nM flavopiridol. Data represent means ± SD from two independent experiments. **P < 0.01.

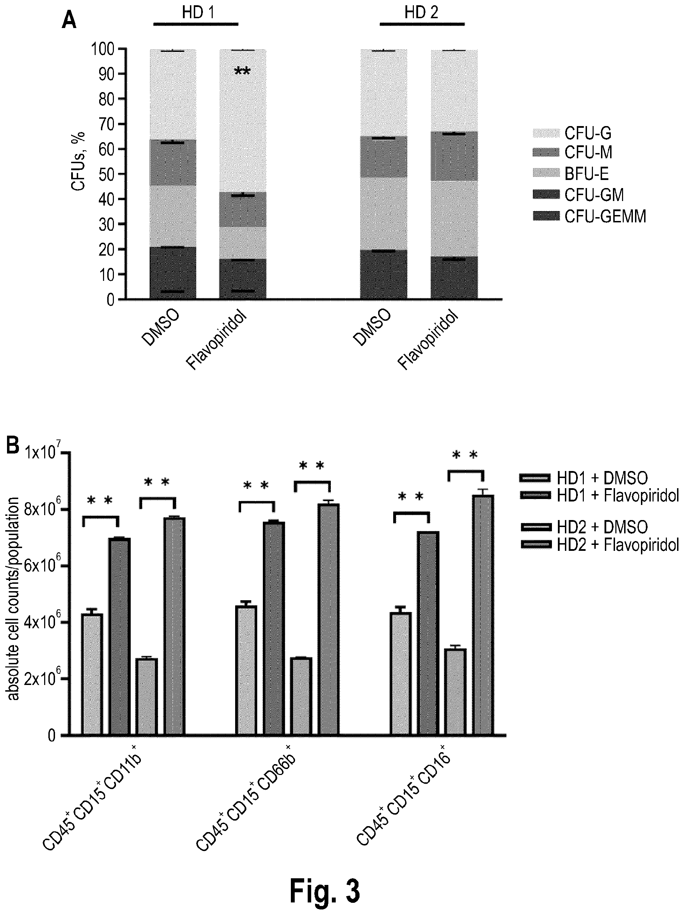

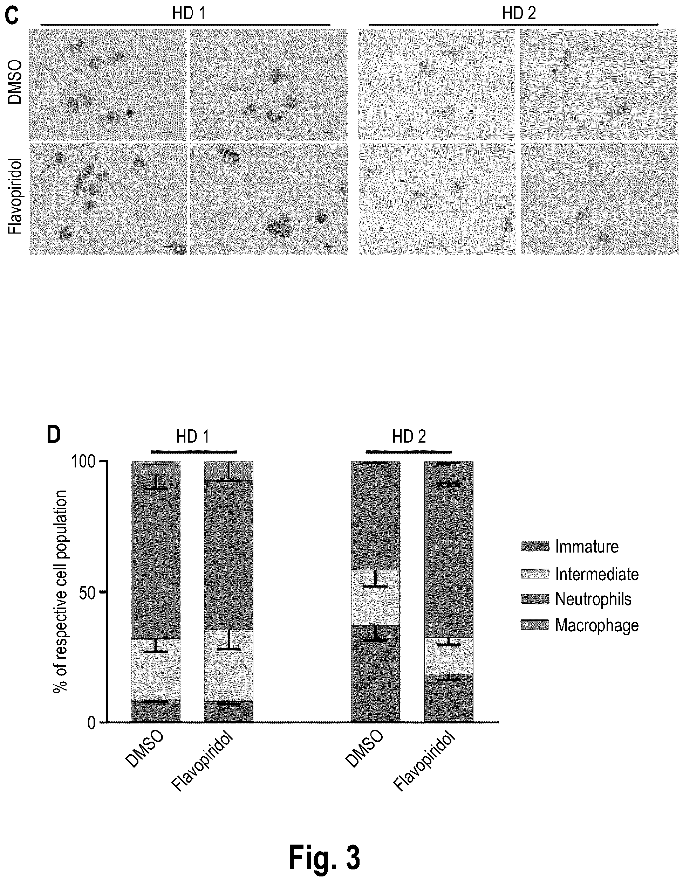

- Fig. 3:: Flavopiridol treatment enhanced granulocytic differentiation of primary bone marrow CD34+ HSPCs of healthy controls. A. Colony-forming unit (CFU) assay of CD34⁺ HSPC cells treated with 40 nM flavopiridol. Data represent means ± SD from two independent experiments. **P < 0.01. B. Differentiation capacity of flavopiridol treated HD-CD34⁺ HSPC cells was assessed by liquid culture differentiation after 14 days by investigating neutrophilic surface marker expression. Data represent means ± standard deviation (SD) from duplicates. **P<0.01. C-D. Wright-Giemsa staining of differentiated cells was conducted on day 14 allowing morphologic discrimination of the cells. Representative images are depicted.

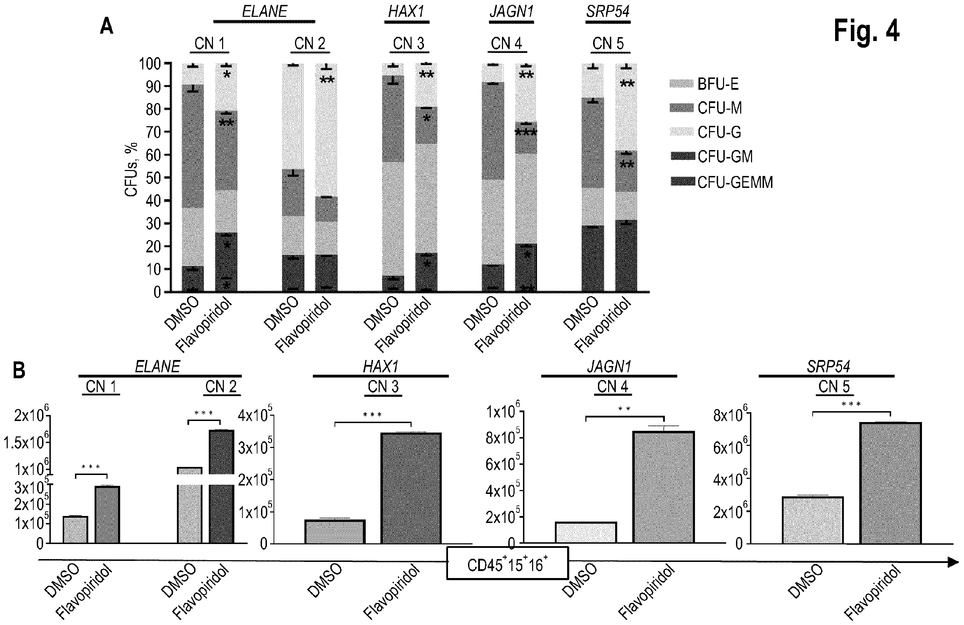

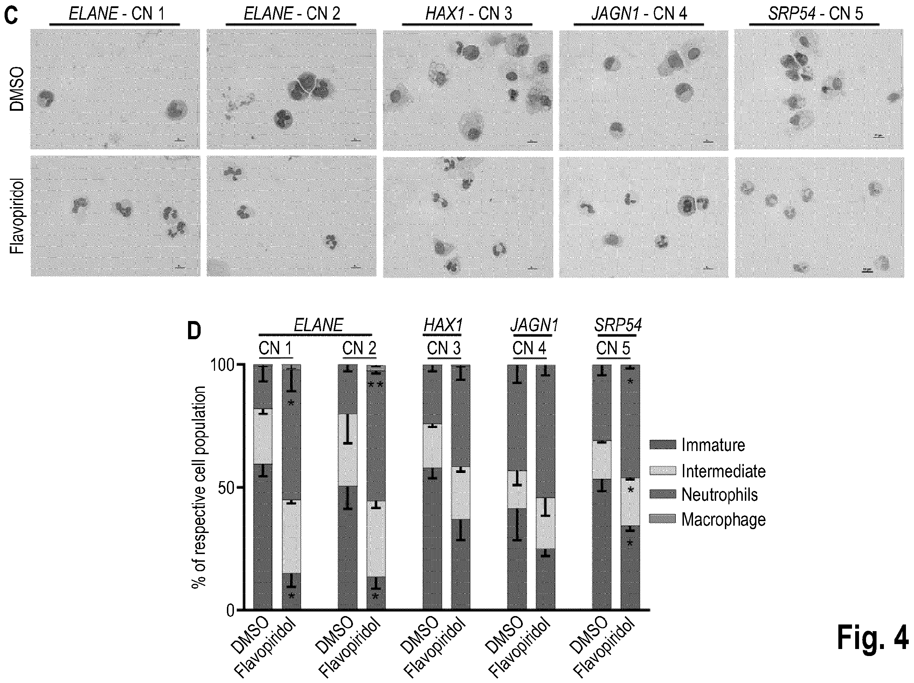

- Fig. 4:: Flavopiridol treatment restored granulocytic differentiation of primary bone marrow CD34⁺ HSPCs of *ELANE-CN, HAX1-CN, JAGN1-CN* and SRP54-CN patients. A. Colony-forming unit (CFU) assay of CN CD34⁺ HSPC cells treated with 40 nM flavopiridol. Data represent means ± SD from two independent experiments. *P<0.05, **P < 0.01. B. Differentiation capacity of flavopiridol treated CN-CD34⁺ HSPC cells was assessed by liquid culture differentiation after 14 days by investigating neutrophilic surface marker expression. Data represent means ± standard deviation (SD) from duplicates. **P<0.01, ***P<0. 001. C-D. Wright-Giemsa staining of differentiated cells was conducted on day 14 allowing morphologic discrimination of the cells. Representative images are depicted.

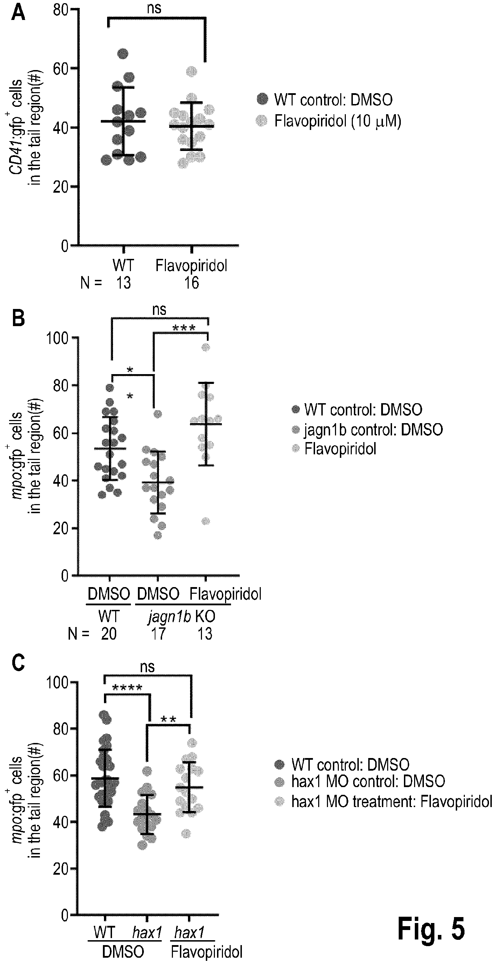

- Fig. 5:: Flavopiridol treatment of transgenic zebrafish mutant embryos restored the number of neutrophils without affecting wildtype HSPCs. A. Flavopiridol treatment did not affect the number of HSPCs (CD41: gfp⁺ cells) when compared to the DMSO-treated controls. B. The jagn1b knock-out embryos showed a reduction of neutrophils (mpo: gfp⁺ cells) when compared to the DMSO treated wildtype controls. Two days of flavopiridol treatment of the jagn1b-knock-out neutropenic embryos restored the number of neutrophils (mpo: gfp⁺ cells) to a similar level than the wildtype controls. C. The numbers of neutrophils (mpo: gfp⁺ cells) were significantly reduced in hax1-knock-down embryos, when compared to the DMSO treated wildtype controls. After two days of flavopiridol treatment, the numbers of neutrophils (mpo: gfp⁺ cells) of hax1-knock-down embryos increased to a similar level than the wildtype controls. Each dot represents an individual embryo. Data are means ± standard deviation. The statistical analysis was performed using an unpaired, two-tailed Wilcoxon-Mann-Whitney test. ns: not significant, * p_value <0.05, ** p_value <0.01, *** p_value <0.001, **** p_value < 0.0001.

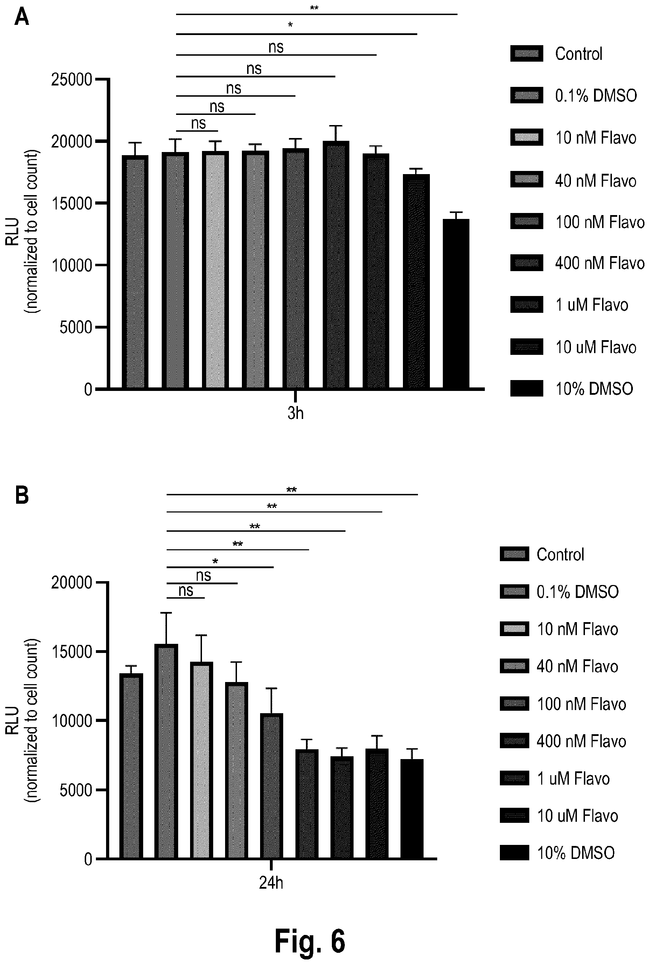

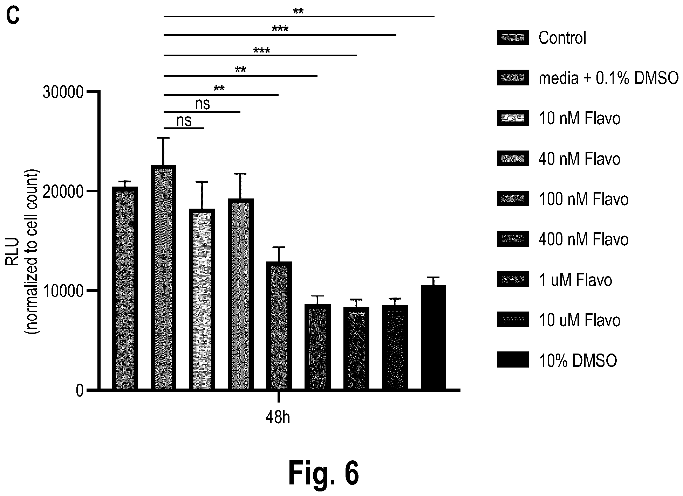

- Fig. 6:: Flavopiridol toxicity on human primary healthy donor neutrophils after treatment for (A) 3 hours, (B) 24 hours, and (C) 48 hours.

### EXAMPLES

### 1. Material and methods

### Patients

Bone marrow and peripheral blood samples were collected from the patients in association with an annual follow-up recommended by the Severe Chronic Neutropenia International Registry. Study approval was obtained from the Ethical Review Board of the Medical Faculty, University of Tübingen. Informed written consent was obtained from the participant of this study.

### Reprogramming of peripheral blood mononuclear cells (PB MNCs)

1.5 × 10⁶ PB MNCs were cultured in Stemline II medium (Sigma) supplemented with 10 % FCS, 1 % penicillin/streptomycin (Biochrom, #A2213), 1 % glutamine (Biochrom, #K0283) and cytokines: 20 ng/µl IL-3 (PeproTech, #200-03), 20 ng/µl II-6 (PeproTech, #200-06), 20 ng/µl TPO (R&D Systems, #288-TP-200), 50 ng/µl SCF (R&D Systems, #255-SC-200) and 50 ng/µl FLT-3L (BioLegend, #550604) for 7 days. On day 7, cells were transferred to Retronectin (Clontech)-coated 12-well plates and lentiviral supernatants with Oct3/4, Klf4 and Sox2 cDNAs (pRRL.PPT.SF.hOct34.hKlf4.hSox2.i2dTomato.pre.FRT kindly provided by A. Schambach) with a multiplicity of infection (MOI) of 2. After 4 days, cells were transferred to SNL-feeder cells and cultured in the mixture of a half of iPSCs- maintenance medium DMEM F12 (Sigma, #D6421-6x) supplemented with 20 % knock out serum replacement (Invitrogen, #10828028), 1 % non-essential amino acids solution (Invitrogen, #11140-050), 100 uM (2-Mercaptoethanol and 2mM L-Glutamine) and a half of CD34⁺ cells expansion medium supplemented with 2 mM valproic acid and 50 µg/ml vitamin C. Medium was gradually changed to iPSCs medium only. First iPSCs colonies appeared approximately three weeks after initiation of reprogramming.

### Design of the ELANE-specific guide RNA (gRNA)

gRNA for correction of the ELANE gene mutations p. A57V (cut site: chr19 [CTGCGCGGAGGCCACTTCTG (SEQ ID NO:1), +852,969: -852,969], NM_001972.3 Exon 2, mutations p. C151Y (cut site: chr19 [GGGACGCCGCCTGGGCAACG (SEQ ID NO:2), +855,637: -855,637], NM_001972.3 Exon 4, mutations p. G214R (cut site: chr19 [TTAGCCCGTTGCAGACCAAG (SEQ ID NO:3), +855,978: -855,978], NM_001972.3 Exon 5 were designed using the CCTop webtool.

### CRISPR/Cas9 based Repair template

Repair templates for ELANE mutations p. A57V (5'-CGGCCCCACGCGTGGCCCTTCATGGTGTCCCTGCAGCTGCGCGGAGGCCACTTCTGC GGCGCCACCCTGATTGCGCCCAACTTCGTCATGTCGGCCGCGCACTGCGTGGCGAATG TGTGAGTA-3' (SEQ ID NO:4)), p.C151Y (5'-GCCAACGTGCAGGTGGCCCAGCTGCCGGCTCAGGGACGCCGCCTGGGCAACGGGGT GCAGTGCCTGGCCATGGGCTGGGGCCTTCTGGGCAGGAACCGTGGGATCGCCAGCGT CCTGCAGGAG-3' (SEQ ID NO:5)) and p. G214R (5'-CGGGGCAAAGGCATCGGGGTAGAGCCCTGAGGCGCAGCCTCCCCGGACGAAGGAGG CAATTCCGTGGATTAGCCCGTTGCAGACCAAGGGGCTGCCGGAGTCCCCCTGTGGAGG CAGACAAGG-3' (SEQ ID NO:6)) were designed based on human genome assembly GRCh37 (hg19) and synthesized as ssODN by Integrated DNA technologies (IDT).

### CRISPR/Cas9-gRNA RNP mediated ELANE mutation correction in CN-iPSCs

Electroporations were carried out using the Amaxa nucleofection system (P3 primary kit, #V4XP-3024) according to the manufacturer's instructions. 1 × 10⁶ human CN-iPSCs were electroporated with assembled gRNA (8 µg) and HiFi Cas9 (15 µg) protein (Integrated DNA Technologies) along with 1 µM respective repair template.

### Isolation of single cell iPSCs clones

8 × 10³ human iPSCs were plated on Geltrex-coated 10-cm dish in StemFlex medium (Thermo Fisher Scientific, #A3349401) and RevitaCell supplement (Thermo Fisher Scientific, #A2644501). The medium was changed every 24 hours without RevitaCell supplement. On day 7, single iPSC colonies were picked and transferred to the Geltrex-coated 96-well plates (one clone/well).

### Screening of single iPSCs clones

Genomic DNA was isolated from single iPSC clones using the QuickExtract DNA extraction kit (Lucigen, #QE09050). PCR was carried out using the GoTaq Hot Start Polymerase Kit (Promega, #M5006) and ELANE-specific primers (Table 1).

**Table 1. ELANE-specific PCR primers.**

| Primer name | Sequence (5' to 3') | Amplified region |
|---|---|---|
| ELANE_Exon2_F | GGCTCCTTGGCAGGCACTCA (SEQ ID NO:7) | Exon 2 |
| ELANE_Exon2_R | CACCTCACAGACCGGGACGC (SEQ ID NO:8) | Exon 2 |
| ELANE_Exon4_F | CGCCCTGAGCCTTGGTGACG (SEQ ID NO:9) | Exon 4 |
| ELANE_Exon4_R | AGCCACGGTGCCTGTTGCTG (SEQ ID NO:10) | Exon 4 |
| ELANE_Exon5_F | CAGCCCGGACTGCAGCAACA (SEQ ID NO:11) | Exon 5 |
| ELANE_Exon5_R | GACCCGGGCAGCCCTTCTCA (SEQ ID NO:12) | Exon 5 |

*In vitro* restriction enzymatic (RE) digestion of PCR products was performed to exclude the clones that do not show the correction RE pattern. The reaction mix was incubated at 37°C for 60 minutes and subsequently run on 1% agarose gel. The PCR products from iPSC clones that showed corrected RE pattern were purified by ExoSAP, which is a master mix of one part of Exonuclease I 20 U/µl (Thermo Fisher Scientific, #EN0581) and two parts of FastAP thermosensitive alkaline phosphatase 1U/µl (Thermo Fisher Scientific, #EF0651). Sanger sequencing of purified PCR products was performed by Eurofins Genomics or Microsynth. Analysis of CRISPR/Cas9 based genome editing performed by the tracking of Indels by Decomposition (TIDE) or Inference of CRISPR Edits (ICE) webtools.

### EB-based hematopoietic differentiation of iPSCs

iPSCs were cultured on SNL feeder cells for 24 hours and then dissociated using PBS/EDTA (0.02 %) for 5 min. EB generation was performed by centrifugation of 2 × 10⁴ cells per EB in 96-well plates using APEL serum-free differentiation medium (Stemcell Technologies) supplemented with bFGF (20 ng/µl) and ROCK Inhibitor (R&D). On day one, BMP4 (40 ng/µl) was added to the culture medium to induce mesodermal differentiation. On day four, EBs were plated on Matrigel-coated 6-well plates (10 EBs/well) in APEL medium supplemented with VEGF (40 ng/µl), SCF (50 ng/µl) and IL-3 (50 ng/µl). For neutrophilic differentiation, the medium was changed 3 days later to fresh APEL medium supplemented with IL3 (50 ng/µl) and G-CSF (50 ng/µl). All cytokines were purchased from R&D Systems if nothing else is indicated. First hematopoietic suspension cells appeared between day 10 and 14. Suspension cells were harvested every 3 - 4 days and analyzed starting from day 14 to day 32. For flow cytometry analysis, cells were incubated in PBS/1% BSA containing 0.05 % sodium azide and stained with mouse monoclonal anti-human antibodies. Two multicolor flow cytometry antibody panels were applied: 'early-stage' hematopoietic differentiation panel using CD33 (BioLegend, #303416), CD34 (BD, #348811), CD309 (BioLegend, #359910), CD43 (BD, #560199), CD41a (BD, #557296), CD235a (BD, #559943), CD45 (BioLegend, #304036), 7-AAD (BD, #559925); and 'late-stage' myeloid/hematopoietic differentiation using CD15 (BD, #555402), CD45 (BioLegend, #304012), CD33 (BioLegend, #303416), 7-AAD (BD, #559925). For iPSC characterization, antibodies against human TRA1-60 (BD, #560380), mouse TRA-1-85/CD147 (R&D, #FAB3195A) and human SSEA4 (BD, #560126) were used. Anti-Mouse Ig κ/Negative Control Compensation Particles (BD Biosciences) were applied for compensation. Samples were analyzed on FACSCanto II (BD) and FlowJo V10 (FlowJo LLC).

### Liquid culture differentiation of CD34⁺ cells

CD34⁺ cells were seeded in a 24-well plate at a density of 2 × 10⁵ cells/ml. Cells were incubated for 7 days in RPMI 1640 GlutaMAX supplemented with 10 % FBS, 1 % penicillin/streptomycin, 5 ng/ml SCF, 5 ng/ml IL-3, 5 ng/ml GM-CSF and 10 ng/ml G-CSF. Medium was exchanged every second day. On day 7, cells were plated in RPMI 1640 GlutaMAX supplemented with 10 % FBS, 1 % penicillin/streptomycin and 10 ng/ml G-CSF. On day 14, cells were analyzed by flow cytometry using following mouse anti-human antibodies: CD34 (BD, #348811), CD33 (BioLegend, #303416), CD45 (BioLegend, #304036), CD11b (BD, #557754), CD15 (BD, #555402), CD66b (BioLegend, #305114), CD16 (BD, #561248).

### Zebrafish maintenance and breeding

Zebrafish transgenic lines and wild type were maintained according to standard protocols and handled in accordance with European Union animal protection directive 2010/63/EU. The maintenance was approved by the local government (Tierschutzgesetz §11, Abs. 1, Nr. 1, husbandry permit 35/9185.46/Uni TÜ). All the experiments were performed in zebrafish embryos under 5 days post fertilization. In this study the inventors used two transgenic (tg) reporter lines, tg(mpo: gfp), where neutrophils express gfp and tg(CD41:gfp),where HSPCs express gfp, both described in the art.

### Microinjection and drug treatment

To generate the jagn1b and hax1 mutants two different approaches were used, CRISPR/Cas9-mediated knock-out and morpholino-based knock down, respectively. On the one hand, to interfere with the function of jagn1b, jagn1b-targeting sgRNA and Cas9 protein RNPs (Alt-R S.p. Cas9 nuclease, v.3, IDT) were injected into the blastomere of 1-cell stage tg (mpo: gfp) embryos, as described in the art. On the other hand, to interfere with hax1 function 1 mM hax1-specific morpholino (GeneTool) was injected into 1-cell stage tg(mpo:gfp) embryos as previously described in the art. All mutant and uninjected control embryos were raised until one day post-fertilization and dechorionized with pronase (Roche, Cat. No. 10165921001). These embryos where then distributed into 24-well plates, 5embryos/well and treated with either DMSO as control or 5-10µM flavopiridol in E3 zebrafish medium. After two days of treatment, the number of mpo:gfp⁺ or CD41:gfp⁺ cells where quantified using a Nikon stereo-fluorescent SMZ18 microscope.

### 2. Results

First, the inventors established an efficient pipeline for CRISPR/Cas9-based correction of CN-associated inherited gene mutations in CN-derived induced pluripotent stem cells (iPSCs) using CRISPR/Cas9 based genome-editing; Dannenmann et al. (2020), CRISPR/Cas9 genome editing of human-induced pluripotent stem cells followed by granulocytic differentiation. RNA interference and CRISPR technologies: Technical advances and new therapeutic opportunities, pp. 471-483. Using this pipeline, the inventors generated an isogenic counterpart of CN iPSCs (Fig. 1A).

By correcting individual mutations without altering the surrounding factors, e.g., genetic, epigenetic, etc. background, the inventors generated genetically matched non-diseased iPSC controls. Thus, the inventors narrowed down the study solely to the disease-causing mutations. The isogenic iPSC group was differentiated toward neutrophils *in vitro* and characterized by flow cytometry-based analysis of cell surface markers.

Comparison of myeloid differentiation between isogenic iPSC lines (ELANE-CN corr) and respective parent iPSC lines (ELANE-CN) proved successful restoration of the granulocytic cell production in the iPSC lines expressing the corrected *ELANE* gene (Fig. 1B).

These data clearly indicated that the inventors successfully established an isogenic iPSC-based model of CN.

Subsequently, the inventors performed transcriptome profiling of isogenic hematopoietic stem and progenitors (HSPCs) to extract the differentially expressed genes (DEGs) between CN and CN-corrected hematopoietic progenitor cells. The differentially expressed gene signature considered the difference between hematopoietic progenitor cells derived from CN iPSCs and their corrected counterparts in isogenic setting. The differentially expressed gene signature was submitted to LINCS L1000 characteristic direction signatures search engine (L1000CDS), which is a publicly available database comprised of over a million gene expression profiles of chemically perturbed human cell lines.

The inventors then selected eleven small molecules that could potentially mimic the differentially expressed genes (DEGs) signature between CN and CN-corrected HSPCs and screened the effect of these small molecules on granulopoiesis using their isogenic CN iPSC model. The screening results showed that the CDK2/4/9 inhibitor flavopiridol markedly restored the granulopoiesis in CN iPSCs.

Upon the screening results, flavopiridol treatment was applied on three independent *ELANE-CN* derived iPSCs during granulopoiesis differentiation in the setting of the colony-forming unit assay (CFU). Interestingly, the inventors observed a restoration of granulopoiesis in CN iPSCs according to a significant increase of colony-forming unit-granulocytes (CFU-G) and a significant decrease of CFU-macrophages (CFU-Ms) (Fig. 2).

To investigate whether flavopiridol disturbs the granulopoiesis process of healthy individuals, the inventors treated primary bone marrow CD34⁺ HSPCs of healthy donors with 40 nM flavopiridol and performed CFU assay as well as *in vitro* granulocytic liquid culture differentiation (LCD) assays. The CFU assay showed significantly elevated levels of CFU-G in healthy donor one (HD1) and no significant change in healthy donor two (HD2) when compared to DMSO control (Fig. 3A).

The differentiation capacity of flavopiridol treated CD34⁺ HSPCs was assessed by liquid culture differentiation for 14 days, followed by flow cytometry analysis of neutrophilic surface markers. The inventors observed significantly enhanced granulopoiesis in both healthy CD34⁺ HSPCs upon flavopiridol treatment as numbers of granulocytes (CD45⁺CD15⁺CD11b⁺ and CD45⁺CD15⁺CD66b⁺) and mature neutrophils (CD45⁺CD15⁺CD16⁺) increased (Fig. 3B). At the same time, morphological examination of cytospin preparations of mature granulocytes generated on day 14 of the *in vitro* granulocytic liquid culture differentiation (Fig. 3C) showed no change in healthy donor one (HD1) and a significant increase in neutrophil count in healthy donor two (HD 2) (Fig. 3D).

Taken together, the results of the inventors showed flavopiridol not only did NOT interrupt granulopoiesis, but also enhanced it in primary bone marrow CD34⁺ HSPC of healthy donors.

To further evaluate the clinical applicability of flavopiridol as a universal treatment option for all CN patients regardless of the underlying genetic cause, the inventors treated primary bone marrow CD34⁺ HSPCs of two *ELANE-CN* patients, one *HAX1*-CN, one *JAGN1*-CN and one *SRP54*-CN patient with 40 nM flavopiridol and performed CFU and LCD assays. The CFU assay showed significantly elevated levels of CFU-G in all CN groups (Fig. 4A).

Differentiation capacity of flavopiridol treated CD34⁺ HSPCs was assessed by liquid culture differentiation for 14 days, followed by flow cytometry analysis of neutrophilic surface marker expression of absolute population. The inventors observed a significant restoration of granulopoiesis in all CD34⁺ HSPCs of CN patients upon flavopiridol treatment as numbers of granulocytes (CD45⁺CD15⁺CD11b⁺ and CD45⁺CD15⁺CD66b⁺) and mature neutrophils (CD45⁺CD15⁺CD16⁺) were restored (Fig. 4B).

At the same time, morphological examination of cytospin preparations of mature granulocytes generated on day 14 of the *in vitro* granulocytic liquid culture differentiation (Fig. 4C) showed a significant restoration or restoration trend of granulopoiesis in all CN patients (Fig. 4D).

Taken together, the results of the inventors generated by their CN *in vitro* models showed that flavopiridol restored granulopoiesis in primary bone marrow CD34⁺ HSPCs of *ELANE-CN, HAX1*-CN, *JAGN1-CN* and SRP54-CN patients.

To further validate the results generated from the *in vitro* models of the inventors, they have designed a set of experiments and studied the effect of flavopiridol in zebrafish *in vivo* model. Up to 10 µM flavopiridol treatment did not affect the total number of HSPCs (CD41: gfp⁺ cells) when compared to the DMSO-treated controls (Fig. 5A).

Two days flavopiridol treatment of transgenic jagn1b knock-out zebrafish mutant embryos -representing *in vivo JAGN1-CN* model- significantly restored the number of neutrophils (mpo: gfp⁺ cells) to a similar level to wildtype controls (Fig. 5B). Two days flavopiridol treatment of hax1-knock-down zebrafish mutant embryos (hax1 MO) - representing *in vivo hax1*-CN model- significantly restored the number of neutrophils (mpo: gfp⁺ cells) to a similar level to wildtype controls (Fig. 5C).

The results generated in zebrafish model for hax1 or jagn1 associated congenital neutropenia are cross-validating the results generated in the inventors' CN-iPSCs or CN HSPCs *in vitro.*

To study the toxicity effect of Flavopiridol - in a dose-dependent manner - on human primary neutrophils, the inventors have performed RealTime-Glo MT Cell Viability Assay (Promega # G9711). The assay measures the reducing potential of viable cells in an ATP-independent manner and the nonlytic nature, high sensitivity and rapid response of the assay make it possible to track the cell viability over time in the same plate.

Ficoll-Paque density gradient media (density: 1.077 g/mL) used for isolation of peripheral blood polymononuclear cell (PBPMNs)1 of healthy donors. Human primary neutrophils of healthy donor treated with a wide concentration range of Flavopiridol (10 nM to 10 µM) and cell viability measured at 3, 24 and 48 hours post treatment in the same samples according to manufacturer's protocol using Glomax plate reader.

No significant difference observed in viability of healthy human primary neutrophil treated with 40 nM Flavopiridol for 3 hours (Fig. 6A), 24 hours (Fig. 6B) and 48 hours (Fig. 6C) when compared with neutrophils treated with 0.1% DMSO.

Overall, these data define a new field of application for CDK2/4/9 inhibitors, exemplarily demonstrated by flavopiridol, where they can be used to restore granulopoiesis for different genetic groups of neutropenia patients.

### 3. Conclusion

Taken together, the findings of the inventors showed that a CDK2/4/9 inhibitor restores granulopoiesis in CN patients while enhancing it in healthy donors. These results will lead to the establishment of a new treatment not only for CN patients but also neutropenia patients in general.

## Claims

1. A cyclin-dependent kinase 2/4/9 (CDK2/4/9) inhibitor and salts thereof, solvates thereof and solvates of the salts thereof, for use in the prophylaxis and/or treatment of neutropenia.

2. The CDK2/4/9 inhibitor of claim 1, which is selected from the group consisting of: flavopiridol (alvocidib), rohitukine, TP-1287, seliciclib (roscovitine/CYC202), dinaciclib (SCH 727965), SNS 032, P276-00, AT7519, voruciclib, CDKI-73, TG02, BAY1143572 (atuveciclib), BAY1251152, AZD4573, i-CDK9, and NVP-2.

3. The CDK2/4/9 inhibitor of claim 1 or 2, wherein said neutropenia is any selected from the group consisting of: congenital and/ or acquired neutropenia, preferably severe congenital neutropenia (CN/SCN), cyclic neutropenia (CyN), chronic neutropenia, including idiopathic neutropenia, drug-induced neutropenia, immune neutropenia, acute neutropenia caused by chemotherapy and/or radiation.

4. The CDK2/4/9 inhibitor of any of the preceding claims, which is flavopiridol (alvocidib).

5. The CDK2/4/9 inhibitor according to any one of the preceding claims, wherein the inhibitor is used at a dosage which, after administration into a living being, results in blood plasma concentrations which are in the area of approx. 10 - approx. 300, preferably approx. 20 - approx. 200, further preferably approx. 30 - approx. 100, and most preferably approx. 40 nM.

6. The CDK2/4/9 inhibitor of any of the preceding claims, wherein the neutropenia is severe congenital neutropenia (CN/SCN) or cyclic neutropenia (CyN).

7. The CDK2/4/9 inhibitor of any of the preceding claims, wherein the use is in the prophylaxis and/or treatment of neutropenia patients who do not respond or do not respond satisfactorily to treatment with granulocyte colony-stimulating factor (G-CSF).

8. The CDK2/4/9 inhibitor of claim 7, wherein the G-CSF is recombinant human G-CSF (rhG-CSF).

9. The CDK2/4/9 inhibitor of any of the preceding claims for use in combination with G-CSF.

10. The CDK2/4/9 inhibitor of claim 9, wherein the G-CSF is rhG-CSF.

11. A pharmaceutical composition for use in the prophylaxis and/or treatment of neutropenia comprising the CDK2/4/9 inhibitor and the salts thereof, the solvates thereof and the solvates of the salts thereof according to any of claims 1-8 and a pharmaceutically acceptable carrier.

12. The pharmaceutical composition of claim 11, further comprising G-CSF.

13. The pharmaceutical composition of claim 12, wherein the G-CSF is rhG-CSF.

14. A method for the prophylactic and/or therapeutic treatment of neutropenia in a living being, comprising the administration of an effective amount of the CDK2/4/9 inhibitor and the salts thereof, the solvates thereof and the solvates of the salts thereof according to any of claims 1-8, or the pharmaceutical composition of any of claims 11-13 to said living being.
